# EUROPÄISCHE PATENTSCHRIFT

(11) **EP 1 311 200 B1**
(45) Veröffentlichungstag und Bekanntmachung des Hinweises auf die Patenterteilung: **26.05.2004**
(21) Anmeldenummer: 01974112.3
(22) Anmeldetag: 04.08.2001
(51) Int. Cl.: A61B 18/14

(54) **UROLOGISCHES RESEKTOSKOP MIT KONTAKTIEREINRICHTUNG**
UROLOGICAL RESECTOSCOPE COMPRISING A CONTACTING DEVICE
RESECTOSCOPE UROLOGIQUE AVEC DISPOSITIF DE MISE EN CONTACT

(30) Priorität: 26.08.2000 DE 10042095
(43) Veröffentlichungstag der Anmeldung: 21.05.2003
(73) Patentinhaber: OLYMPUS WINTER & IBE GMBH, 22045 Hamburg (DE)
(72) Erfinder: BROMMERSMA, Pieter, 22941 Bargteheide (DE); NUSSBAUM, Felix, 22089 Hamburg (DE); WOSNITZA, Thomas, 21337 Lüneburg (DE)
(74) Vertreter: Schaefer, Konrad, Dipl.-Phys.
(86) Internationale Anmeldenummer: PCT/EP2001/009043
(87) Internationale Veröffentlichungsnummer: WO 2002/017806

(56) Entgegenhaltungen:
- US-A- 4 919 131
- US-A- 6 105 581

## Beschreibung

Die Erfindung betrifft ein Resektoskop der im Oberbegriff des Anspruch 1 genannten Art.

Gattungsgemäße Resektoskope sind in erster Linie für die Prostataresektion vorgesehen, jedoch ihrer Bauart nach gegebenenfalls auch für andere chirurgische Einsätze verwendbar. Unter Resektoskop werden hier endoskopische Geräte verstanden, bei denen in einem Schaftrohr eine Optik und ein Elektrodenträger mit distaler Elektrode angeordnet sind, wobei der Elektrodenträger mit der Elektrode in Achsrichtung verschiebbar gelagert ist und mit seinem proximalen Ende an einem Schiebekörper des Resektoskopes befestigt und elektrisch kontaktiert ist, welcher mit einer Griffbetätigung von Hand axial verstellt werden kann, um die Elektrode axial zu verschieben.

Bei der Prostataresektion wird das Resektoskop mit dem distalen Ende des Schaftrohres durch die Harnröhre bis in das Innere der Prostata vorgeschoben. Bei eingeschalteter HF-Beaufschlagung der Elektrode kann diese durch Handbewegung des Schiebekörpers, an dem sie proximal befestigt ist, vor- und zurückgeschoben werden, um Gewebe zu schneiden. Die Elektrode ist dabei üblicherweise als Drahtschlinge ausgebildet, mit der Gewebeschnipsel resiziert werden können. Die Elektrode kann auch anders ausgebildet sein, beispielsweise als Knopfelektrode, Rollenelektrode, Messerelektrode oder dergleichen, um für unterschiedliche Einsatzzwecke z. B. Koagulieren, Schneiden und dergleichen verwendet werden zu können.

Probleme bildet dabei stets die ordnungsgemäße Kontaktierung des die Elektrode stromversorgenden Leiterdrahtes an seinem Kontaktabschnitt im Schiebekörper. Dort muß der Kontakt mit einem weiterführenden Kabel hergestellt werden, das zu einem getrennt aufgestellten HF-Generator führt.

Bei älteren Konstruktionen wurde im Schiebekörper mit einer Klemmschraube gleichzeitig kontaktiert und die mechanische Befestigung des Elektrodenträgers sichergestellt. Verschmort diese Kontaktierungsstelle, so muß der gesamte Schiebekörper ausgewechselt werden.

Eine gattungsgemäße Konstruktion ist aus US 4,917,621 und US 4,919,131, jeweils in Fig. 3 bekannt. Der Schiebekörper weist einen quer durchgehenden Schacht auf, in den der Stecker des weiterführenden HF-Kabels zur Kontaktierung des im Schacht frei liegenden Kontaktabschnitts des Elektrodenträgers einsteckbar ist. Distal vom Schacht ist eine an einem Befestigungsabschnitt des Elektrodenträgers angreifende Klemmeinrichtung vorgesehen.

Vorteilhaft an dieser Konstruktion ist die Möglichkeit, den Elektrodenträger mit der Klemmeinrichtung am Schiebekörper gesondert mechanisch zu befestigen, so daß seine ordnungsgemäße mechanische Funktion zunächst ausprobiert werden kann. Anschließend kann mit dem Stecker kontaktiert werden. Verschmort die Kontaktierungsstelle, so muß nur der Elektrodenträger und das Kabel mit dem Stecker gewechselt werden. Die Klemmeinrichtung und der Schiebekörper bleiben unversehrt, da die Klemmeinrichtung gesondert ausgebildet ist.

Nachteilig bei der bekannten gattungsgemäßen Konstruktion ist die strenge Führung des Steckers im Schacht, die dem vom Stecker abgehenden Kabel eine festgelegte Lage aufzwingt. Dies ist bei Resektoskopen von Nachteil, da diese während des Arbeitens zum Beispiel in der Prostata laufend gedreht werden müssen, um mit der Elektrode Schnitte unter unterschiedlichen Winkeln durchführen zu können. Dabei stört das in fester Winkelstellung vom Resektoskop abgehende HF-Kabel.

Aufgabe der Erfindung ist es, ein gattungsgemäßes Resektoskop mit bezüglich des HF-Kabelabganges verbesserten Eigenschaften zu schaffen.

Diese Aufgabe wird mit den Merkmalen des Anspruches 1 gelöst.

Erfindungsgemäß weist die Kontaktiereinrichtung eine Durchbrechung auf, die die Aufnahme nach drei Seiten völlig frei legt. Der in der Aufnahme sitzende Elektrodenträger ist also in der Durchbrechung unter einem Umfangswinkel von wenigstens 180° allseitig frei erreichbar. Der Stecker ist als Klemmstecker ausgebildet, der selbsthaltend schwenkbar auf dem Kontaktabschnitt des Elektrodenträgers greift und innerhalb der Durchbrechung unter einem größeren Winkel von bis über 180° um die Achse des Elektrodenträgers schwenkbar ist. Damit kann bei Drehung des Endoskopes das am Stecker hängende Kabel pendelnd nach unten hängen und behindert die Drehbewegung nicht mehr.

Vorteilhaft sind die Merkmale des Anspruches 2 vorgesehen. Auf diese Weise wird die sichere Klemmung des Klemmsteckers verbessert, da dieser mit der Nut sicheren Halt auf dem Elektrodenträger gegen Abreißen findet.

In den Zeichnungen ist die Erfindung beispielsweise und schematisch dargestellt.

Es zeigen:
- Fig. 1: einen Längsschnitt durch ein erfindungsgemäßes Resektoskop und
- Fig.2: einen Schnitt nach Linie 2 - 2 in Fig. 1.

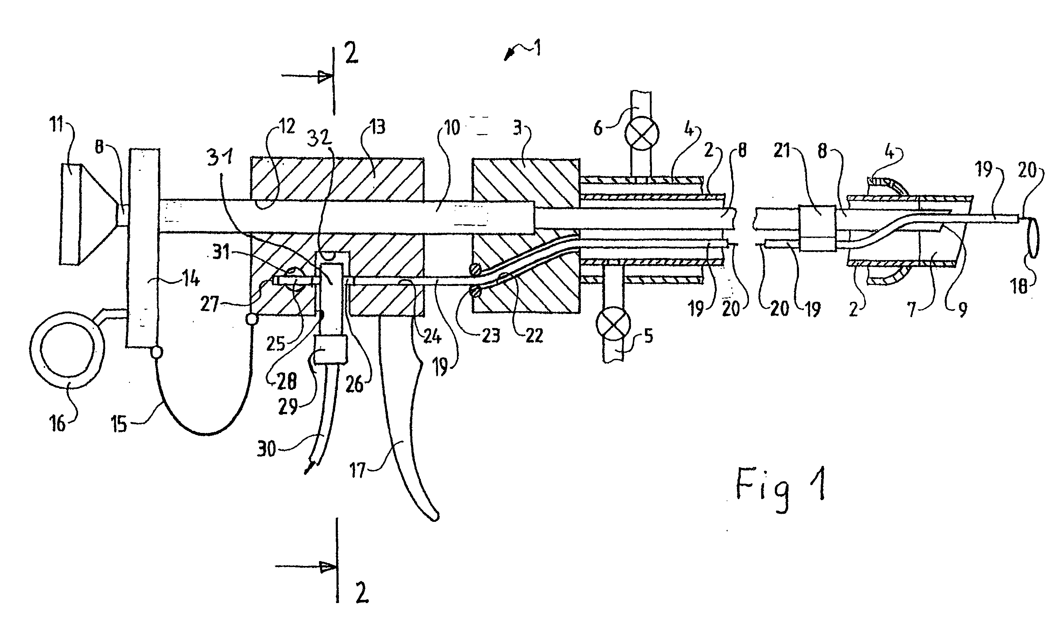

Das in Fig. 1 dargestellte Resektoskop 1 weist ein Schaftrohr 2 auf, das mit seinem proximalem Ende an einem Hauptkörper 3 befestigt ist. In nicht dargestellter Weise kann das Schaftrohr 2 mit der üblichen Kupplungseinrichtung abnehmbar am Hauptkörper 3 befestigt sein. Um das Schaftrohr 2 herum ist ein Außenrohr 4 vorgesehen, das ebenfalls am Hauptkörper 3 befestigt ist und zwar ebenfalls in der üblichen Weise mit einer nicht dargestellten Kupplung. Das Innere des Schaftrohres 2 dient in üblicher Weise als Zulaufkanal für die Dauerspülung und ist, wie die Fig. zeigt, von außen über einen mit Ventil versehenen Anschluß 5 erreichbar, an den ein Schlauch anschließbar ist. Ein weiterer gleichartiger Anschluß 6 zum Anschließen eines weiteren Schlauches ist an den Ringspalt zwischen Schaftrohr 2 und Außenrohr 4 angeschlossen, der als Rücklaufkanal dient.

Die beiden Rohre 2, 4 bestehen in üblicher Ausbildung aus Metall. Der distale Endabschnitt des Schaftrohres 2 ist in üblicher Weise isolierend, beispielsweise als Keramikendstück 7 ausgebildet.

Im Inneren des Schaftrohres 2 verläuft achsparallel eine Optik 8, die in der dargestellten Montagestellung mit Ihrem distalen Objektiv 9 den Arbeitsbereich vor dem Keramikendstück 7 betrachtet und die proximal den Hauptkörper 3 durchläuft. Sie durchläuft von dort weiter ein im Hauptkörper 3 befestigtes Führungsrohr 10 und endet jenseits von dessen proximalem Ende mit einem Okular 11, an dessen Stelle auch ein Kamera sitzen kann.

Auf dem Führungsrohr 10 ist mit einer Führungsbohrung 12 ein Schiebekörper 13 in axialer Richtung verschiebbar gelagert. Am proximalen Ende des Führungsrohres 10 ist ein Endstück 14 befestigt, demgegenüber der Schiebekörper 13 im dargestellten Ausführungsbeispiel mit der üblichen Blattfeder 15 federnd abgestützt ist. Am Endstück 14 ist ein Daumenring 16 angeordnet, während am Schiebestück 13 ein Fingergriff 17 angeordnet ist. Mit einer Hand des Operateurs kann mit dem Daumen im Daumenring 16 und mit dem Zeigefinger am Fingergriff 17 angefaßt und der Schiebekörper 13 axial bewegt werden. Alternativ kann anstelle der erläuterten "aktiven" Betätigung auch eine "passive" Betätigung vorgesehen sein, bei der die Blattfeder 15 zwischen Schiebekörper 13 und Hauptkörper 3 angeordnet ist und auch die Angriffstellen 16, 17 an diesen Teilen sitzen.

Im dargestellten Resektoskop ist auswechselbar eine HF-beaufschlagbare Elektrode 18 vorgesehen, die in üblicher Ausbildung für die Prostataresektion als rechtwinklig zur Achsrichtung erstreckte Drahtschlinge ausgebildet ist. Die Elektrode 18 wird von einem Elektrodenträger 19 getragen, der als Außenisolierung mit innerem Leiterdraht 20 ausgebildet ist. Der Elektrodenträger 19 ist in üblicher Ausbildung mit einer Hülse 21 längsverschiebbar auf der Optik 8 gelagert und durchläuft das Schaftrohr 2 bis zum Hauptkörper 3. Dort durchläuft er einen seitlich verschwenkten Durchgangskanal 22 mit Ringdichtung 23 zur Flüssigkeitsabdichtung und verläuft von dessen proximaler Mündung unter größerem Achsabstand wiederum parallel zur Achse weiter bis in eine Aufnahmebohrung 24 im Schiebekörper 13. Anstelle der Aufnahmebohrung 24 kann als alternative Ausbildung der Aufnahme für den Elektrodenträger 19 z.B. auch eine nach proximal konisch zulaufende Öffnung, ein zur Seite offener Schlitz oder dergleichen vorgesehen sein.

Im proximalen Endbereich weist der Elektrodenträger 19 einen sein Endstück ausbildenden Befestigungsabschnitt 25 auf, der ausreichend fest z.B. massiv aus Metall ausgebildet ist, um dort den Elektrodenträger sicher mechanisch befestigen zu können. Distal anschließend weist der Elektrodenträger 19 einen Kontaktabschnitt 26 auf, der mit einer elektrisch leitenden Außenfläche versehen ist, welche mit dem Leiterdraht 20 des Elektrodenträger 19 elektrisch leitend verbunden ist.

Die Aufnahmebohrung 24 weist ferner in Form ihres proximalen Endes 27 einen Endanschlag für den Elektrodenträger 19 auf, bis zu dem dieser in proximaler Richtung in die Aufnahmebohrung 24 einsteckbar ist.

Ist der Elektrodenträger 19 in der dargestellten Montagestellung bis zum Endanschlag 27 in die Aufnahmebohrung 24 des Schiebekörpers 13 eingesteckt, so liegt er mit seinem Kontaktabschnitt 26 in einer Durchbrechung 28 des Schiebekörpers 13, in dem der Kontaktabschnitt 26 von außen frei zugänglich ist. Er kann dort mit dem dargestellten Klemmstecker 29 am Ende eines zu einem nicht dargestellten HF-Generators weiterführenden Kabels 30 kontaktiert werden.

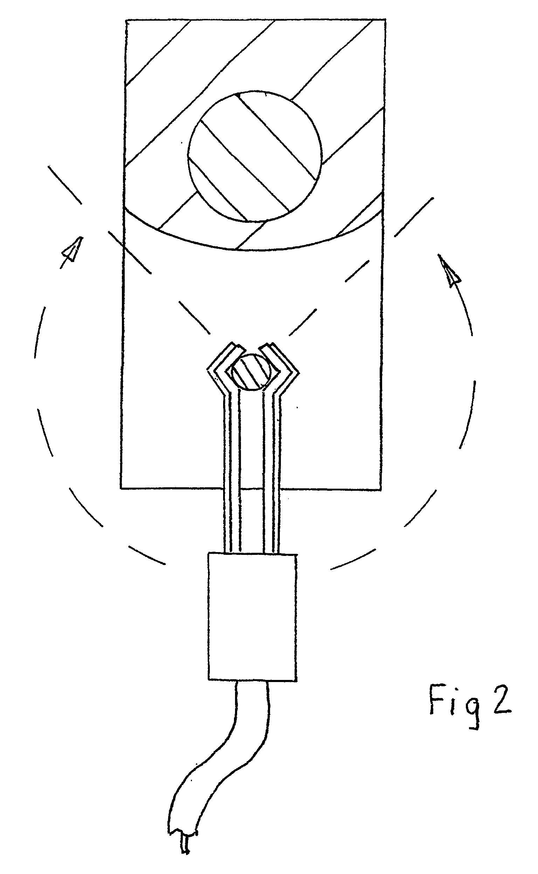

In Figur 2 ist die Form der Durchbrechung 28 näher dargestellt. Man sieht, daß die Durchbrechung 28 den Schiebekörper 13 in einer Richtung quer zum Elektrodenträger 19, der in Figur 2 mit seinem Kontaktabschnitt 26 dargestellt ist, vollständig durchsetzt, und zwar gemäß Figur 2 von links nach rechts. In der dazu lotrechten Querrichtung, also gemäß Figur 2 von unten nach oben, reicht die Durchbrechung von der Unterseite des Schiebekörpers 13 bis zu einer Tiefe jenseits der Aufnahmebohrung, also des darin liegenden Kontaktabschnittes 26, zu einer Fläche 32, die im Abstand zum Kontaktabschnitt 26, also zur Aufnahmebohrung 24, liegt. Damit ist, wie in Figur 2 mit den beiden gestrichelten Linien und dem Kreispfeil dargestellt, der Kontaktabschnitt 26 in der Durchbrechung 28 unter einem Winkel von weit mehr als 180° allseitig zugänglich. Der verbleibende schraffierte Bereich des Schiebekörpers 13 schafft ausreichende Verbindung der in Achsrichtung vor und hinter der Durchbrechung 28 liegenden Teile des Schiebekörpers und ermöglicht ausreichende Schiebeführung des Schiebekörpers 13 mit seiner Führungsbohrung 12.

Der Klemmstecker 29 ist in einem einfachen, für diese Zwecke brauchbaren Ausführungsbeispiel dargestellt. Er weist zwei Backen 31 aus elektrisch leitendem und federndem Material auf, die außen als Berührungsschutz mit Isolierung 33 versehen sind. Wenigstens eine der Backen 31, im Ausführungsbeispiel beide Backen, weisen in ihrem Endbereich Nuten 34 auf, mit denen sie sicher klemmend auf dem Kontaktabschnitt 26 fassen, und zwar mit ausreichend hoher Haltekraft, um das ungewollte Abziehen des Steckers 29 zu verhindern.

Zur Vereinfachung der Zeichnungsdarstellung sind Einrichtungen weggelassen, mit denen die Klemmbacken entgegen ihrer Federkraft geöffnet werden können. Zu diesem Zweck können die Backen 31 beispielsweise am Stecker 29 mit einer nicht dargestellten Öffnungsbetätigungseinrichtung versehen sein. An Stelle die Backen federnd anzuordnen, können sie auch am Stecker 29 gelenkig nach Art von Zangenbranchen gelagert sein mit entsprechender Schließfeder zur Sicherung der Klemmkraft.

Unmittelbar proximal neben der Durchbrechung 28 im Bereich des Befestigungsabschnittes 25 des Elektrodenträgers 19 ist im Schiebekörper 13 eine Befestigungseinrichtung vorgesehen, die im Ausfürungsbeispiel eine Querbohrung 31 aufweist, welche beispielsweise mit Innengewinde zum Einschrauben einer Klemmschraube versehen sein kann. Alternativ kann die Befestigungseinrichtung auch anders ausgebildet sein z.B. mit einem Schieber, der in eine Nut faßt, als Rastverbindung oder dergleichen.

Ist der Elektrodenträger 19 im Schiebekörper 13 ordnungsgemäß befestigt und kontaktiert, so kann durch die beschriebene Schiebebewegung des Schiebekörpers 13 der gesamte Elektrodenträger 19 mit der Elektrode 18 gegenüber dem Schaftrohr 2 längs verschoben werden. Unter Beobachtung durch die Optik 8 kann bei Hochfrequenzbeaufschlagung der Elektrode 18 mit dieser schneidend unter axialer Bewegung gearbeitet werden.

Zum Auswechseln der Elektrode 18 wird der Klemmstecker 29 abgenommen und die Befestigungseinrichtung (Querbohrung 31) gelöst. Dann kann der Elektrodenträger komplett aus dem Resektoskop 1 in distaler Richtung herausgezogen werden. In umgekehrter Weise kann eine neue Elektrode in proximaler Richtung bis zum Endanschlag 27 eingeschoben, mechanisch befestigt und kontaktiert werden. Dabei ist es möglich, zunächst den Elektrodenträger 19 an der Querbohrung 31 mechanisch zu befestigen und die ordnungsgemäße Funktion durch Hin- und Herschieben des Schiebekörpers 13 auszuprobieren, bevor die Kontaktierung mit dem Klemmstecker 29 erfolgt.

Im dargestellten Ausführungsbeispiel trägt der Elektrodenträger 19 eine Elektrode 18 in Form einer üblichen Resektoskopschlinge. Anstelle der Elektrode 18 können auch anders geformte Elektroden vorgesehen sein, wie beispielsweise Knopfelektroden, Stiftelektroden, Rollenelekrtroden oder Elektroden in Messerform, die bei HF-Beaufschlagung koagulierend, vaporisierend oder schneidend arbeiten.

Es können auch bipolare Elektroden verwendet werden, bei denen der Elektrodenträger 19 also zwei an die beiden Pole einer HF-Quelle anzuschließende Elektroden trägt. In diesem Fall sind im Inneren des isolierenden Elektrodenträgers 19 zwei Leiterdrähte 20 vorzusehen. Am proximalen Ende des Elektrodenträgers sind entsprechend anstelle des dargestellten einen Kontaktabschnittes 26 zwei Kontaktabschnitte vorzusehen, die z. B. mit einem Doppelstecker kontaktiert werden können. Die Backen 31 des Klemmsteckers 29 können in diesem Falle z.B. jeweils aus zwei parallelen Federzungen bestehend ausgebildet sein.

Im dargestellten Ausführungsbeispiel ist als Endanschlag für das Einstecken des Elektrodenträgers 19 das Ende 27 der Aufnahmebohrung 24 vorgesehen. Ein Endanschlag kann jedoch auch an der Klemmvorrichtung selbst vorgesehen sein, beispielsweise an der in die Gewindebohrung 31 anzuschraubenden Klemmschraube. Falls als Klemmeinrichtung beispielsweise ein Schieber vorgesehen ist, der in eine Nut auf dem Befestigungsabschnitt 25 eingreift, so kann auch an diesem Schieber ein geeigneter Anschlag vorgesehen sein, der in geeigneter Weise z. B. mit einem entsprechenden Anschlag auf dem Befestigungsabschnitt 25 zusammen wirkt.

## Patentansprüche

1. Urologisches Resektoskop (1) mit einem axial erstreckten Schaftrohr (2), das mit seinem proximalen Ende an einem Hauptkörper (3) befestigt ist, wobei proximal von diesem, gegenüber dem Hauptkörper (3) achsparallel verschiebbar ein Schiebekörper (13) gelagert ist, der eine Aufnahme (24) aufweist, sowie eine Befestigungseinrichtung (31) und einen Klemmstekker (29), wobei in dem Resektoskop (1) eine HF-beaufschlagbare Elektrode (18) mit einem Elektrodenträger (19), der einen außen isolierten Leiterdraht (20) aufweist, axial bis über das distale Ende (7) des Schaftrohres (2) verschiebbar, lagerbar ist, wobei der Elektrodenträger (19) in Montagestellung das Schaftrohr (2) sowie den Hauptkörper (3) bis in die Aufnahme (24) durchlaufend und dort mit der Befestigungseinrichtung (31) befestigbar ist und der Schiebekörper (13) eine ihn quer zur Aufnahme (24) vollständig durchsetzende Durchbrechung (28) aufweist, in die der Klemm-Stecker (29) zur Kontaktierung des Elektrodenträgers (19) eingreift, **dadurch gekennzeichnet, daß** die Durchbrechung (28) in lotrechter Querrichtung zur Aufnahme (24) sich von der Unterseite des Schiebekörpers (13) bis zu einer Tiefe (32) jenseits der Aufnahme (24) erstreckt und daß der Klemmstecker (29) zur schwenkbaren Klemmung auf einem Kontaktabschnitt (26) des Elektrodenträgers (19) ausgebildet ist.

2. Resektoskop nach Anspruch 1, **dadurch gekennzeichnet, daß** der Klemmstecker (29) zwei gegeneinander klemmbare Backen (31) aufweist, von denen wenigstens eine an ihrer Kontaktseite eine quer zur Backe erstreckte Nut aufweist.

## Claims

1. A urological resectoscope (1) with an axially extending shaft tube (2), the proximal end of which is fastened to a main body (3), whereby mounted proximally of it so as to be moveable with respect to the main body (3) parallel to its axis there is a sliding body (13), which has a mounting (24), and a fastening device (31) and a clamping plug (29), whereby an electrode (18), to which HF may be applied, with an electrode carrier (19), which has an externally insulated conductive wire (20), may be mounted in the resectoscope (1) so as to be moveable axially to beyond the distal end (7) of the shaft tube (2), whereby the electrode carrier (19) extends, in the assembled position, through the shaft tube (2) and the main body (3) into the mounting (24) and may be secured there with the fastening device (31) and the sliding body (13) has an opening (28), which passes through completely transversely to the mounting (24) and in which the clamping plug (29) engages to contact the electrode carrier (19), **characterised in that** the opening (28) extends in the perpendicular direction to the mounting (24) from the underside of the sliding body (13) to a depth (32) on the other side of the mounting (24) and that the clamping plug is constructed for pivotable clamping on a contact section (26) of the electrode carrier (19).

2. A resectoscope as claimed in claim 1, **characterised in that** the clamping plug (29) has two jaws (31), which may be clamped against one another and of which at least one has a groove extending transversely to the jaw in its contact surface.

## Revendications

1. Résectoscope urologique (1) comportant une gaine tubulaire (2) axiale allongée fixée par son extrémité proximale à un corps principal (3) du côté proximal duquel est logé un corps coulissant (13) pouvant coulisser par rapport au corps principal (3) et parallèlement à l'axe de celui-ci, ce corps coulissant (13) présentant un logement (24) ainsi qu'un dispositif de fixation (31) et un connecteur à serrage (29) et le résectoscope (1) présentant en son intérieur une électrode (18) pouvant être branchée sur un courant H.F. et un porte-électrode (19) comportant un fil conducteur (20) doté d'une isolation extérieure, cette électrode (18) étant logée de façon à pouvoir être déplacée axialement jusqu'à émerger de l'extrémité distale (7) de la gaine tubulaire (2) et le porte-électrode (19) traversant, en position de montage, la gaine tubulaire (2) ainsi que le corps principal (3) jusqu'au logement (24) où il peut être fixé à l'aide du dispositif de fixation (31), le corps coulissant (13) présentant une ouverture (28) le traversant entièrement en sens transversal par rapport au logement (24) et dans laquelle vient s'enclencher le connecteur à serrage (29) en vue de contacter le porte-électrode (19), **caractérisé en ce que** l'ouverture (28) s'étend, en sens transversal et perpendiculaire au logement (24), de la face inférieure du corps coulissant (13) jusqu'à une profondeur (32) au-delà du logement (24) et que le connecteur à serrage (29) est formé de façon à pouvoir assurer un contact pivotant par serrage au niveau d'une section de contactage (26) du porte-électrode (19).

2. Résectoscope selon la revendication 1, **caractérisé en ce que** le connecteur à serrage (29) présente deux mâchoires de serrage (31) dont l'une au moins présente du côté du contact une gorge s'étendant transversalement par rapport à la mâchoire.
